# EUROPEAN PATENT SPECIFICATION

(11) **EP 0 237 316 B1**
(45) Date of publication and mention of the grant of the patent: **25.08.1993**
(21) Application number: 87302020.0
(22) Date of filing: 10.03.1987
(51) Int. Cl.: A61N 1/05, A61N 1/365, A61N 1/372

(54) **Cardiac pacing device**
Einrichtung zur Herzstimulierung
Dispositif de stimulation cardiaque

(30) Priority: 11.03.1986 US 838607; 19.02.1987 US 16379
(43) Date of publication of application: 16.09.1987
(73) Proprietor: INTERMEDICS, INC., Angleton Texas 77515 (US)
(72) Inventor: Ross, G. Baker Jr, Houston Texas 77098 (US)
(74) Representative: Charlton, Peter John

(56) References cited:
- EP-A- 0 126 981
- DE-A- 2 056 493
- DE-A- 3 300 672
- US-A- 4 573 481
- US-A- 4 677 989
- Journal of the Electrochemical Society, vol.13, no.3, March 1983, pages 731-733 Princeton US; L.S.Robblee et al: "Activated Ir: An electrode suitable for reversible charge injection in saline solution"
- Medical & Biological Engineering & Computing, vol.20, January 1982, pages 77-83, Stevenage GB; F.L.H.Gielen et al: "Comparison of electrode impedances of Pt, Ptlr (10% Ir) and Ir-AIROF electrodes used in electrophysiological experiments"
- PACE, vol.1, October-December 1978, pages 448-457 L-Cammilli et al: "Preliminary experience with the pH-triggered pacemaker"

## Description

The present invention relates generally to artificial cardiac pacing, and more particularly to improved pacing electrodes for stimulating and sensing electrical activity of the heart, and to pacing lead assemblies incorporating such electrodes.

The sinoatrial (S-A) node of the normal mammalian heart acts as the natural pacemaker by which rhythmic electrical excitation is developed and propagated to the atria. In response, the atrial chambers contract, pumping blood into the ventricles. The excitation is propagated through the atrioventricular (A-V) node, which imposes a delay, and then via the conduction system consisting of the bundle of His and Purkinge fibers to the ventricular myocardium, causing contraction and the pumping of blood from the ventricles. Disruption of this natural pacing/propagation system occurs as a result of aging and disease.

Where the human patient has an abnormally slow or abnormally rapid heart rate, or the rate is irregular, it is customary for the cardiologist to prescribe implantation of an artificial cardiac pacemaker selected according to the specific patient's needs. In its simplest form, the cardiac pacemaker consists of a pulse generator with a battery pack, and a lead assembly. The lead assembly includes a pacing electrode to be positioned in stimulating relationship to excitable myocardial tissue, and an insulated electrical coil interconnecting the pulse generator and the pacing electrode to deliver the electrical pulses to the electrode to stimulate the tissue. The electrical circuit is completed via a second electrode (the indifferent or reference electrode), which is connected to a point of reference potential for the cardiac pacemakers, and through the body tissue and fluids. The stimulating electrode may also be used as a sensing electrode by coupling to a detection circuit to sense the electrical activity of the heart. The entire lead/electrode assembly is often referred to simply as the "lead".

In this patent application the pacing electrode is sometimes referred to as the stimulating cathodic electrode, the stimulating electrode, or the cathode, and the indifferent electrode is sometimes referred to as the reference electrode, the anodic electrode, or the anode. It will be understood, however, that electrical activity takes place at each electrode during pacing, and that the coupling may be such that each electrode acts, at different times, as cathode or anode.

The lead of choice for use with the cardiac pacemaker is an endocardial catheter, which is readily inserted transvenously to introduce the stimulating electrode into the cardiac chamber to be paced. In contrast, an epicardial lead requires thoracic surgery to affix the electrode to the surface of the heart. Various forms of active or passive fixation may be employed to maintain the stimulating electrode in proper position relative to the excitable heart tissue, such as sutures (epicardial), a corkscrew or flexible barbs, hooks or tines fastened to the lead in proximity to the electrode.

The cardiac pacemaker may employ unipolar or bipolar stimulation, depending on the preference of the physician and the needs of the patient. For unipolar stimulation, the anode is located remote from the heart, and typically comprises the metal case or portion thereof that houses the batteries, pulse generator and other electronic circuitry of the pacemaker. For bipolar stimulation, the two electrodes are in close proximity, typically with the cathode being at the tip and the anode spaced slightly back from the tip as a ring electrode on the lead.

The cardiac pacemaker may operate in any of several different response modes, including asynchronous, or fixed rate; inhibited, in which stimuli are generated in the absence of specified normal cardiac activity; or triggered, in which the stimuli are delivered in response to specified cardiac activity. In each of these modes, output pulses from the pulse generator are delivered via the lead for electrical stimulation of excitable myocardial tissue at or near the site of the cathode, thereby producing the desired rhythmic contractions of the affected chamber. Since stimulation is attributable to current density, small area stimulating electrodes will suffice. The current required to produce a given current density decreases in direct proportion to the active area of the electrode. Small area cathodic electrodes therefore serve to prolong battery life, and increase the interval between required surgical replacements.

In essence, stimulation requires that the electric field be of sufficient field strength and current density to initiate contraction of excitable myocardial tissue at the cathode site. The minimum electrical impulse necessary to achieve this is referred to as the stimulation threshold. The greater the efficiency of the cathode in impressing the electric field on the tissue, the smaller the amplitude and/or duration of (and the energy contained in) the pacing pulse required to achieve the stimulation threshold. Accordingly, highly efficient, low threshold electrodes conserve energy and prolong battery life. Some authorities have theorized that because greater electrode efficiency lowers the energy required for stimulation, it is a factor in reducing injury to tissue at the stimulation site.

The chronic stimulation threshold for a given patient is typically on the order of two to three times greater than the acute threshold observed at the time of implantation and within the first few days thereafter. The increase in threshold is attributable to fibrotic growth; that is, the formation of a layer of non-excitable tissue about the electrode tip at the stimulation site. This fibrotic layer creates a virtual electrode surface area which is considerably greater than the actual surface area of the electrode, and consequently raises the stimulation threshold. Interestingly, the increase of chronic threshold over acute threshold is proportionately greater (to a limit) as electrode area is decreased, presumably because the ratio of virtual to actual surface area is higher for small area electrodes. Many authorities have speculated that the particular composition of the electrode may contribute to or retard fibrotic growth.

Cardiac pacing may be achieved with anodal rather than cathodal stimulation, but the stimulation threshold is higher because the polarizing force of the stimulating electric field on ions at the surface of membranes of the excitable myocardial cells reduces transmembrane potential on the side of each affected cell furthest from the anode, at a point of relatively lower field intensity. This is precisely opposite to the action that occurs with cathodal stimulation, and results in the higher threshold for anodal stimulation.

Regardless of the type of pacemaker implanted, from the simple fixed rate device to the complex dual chamber pacing/sensing devices and the latest physiologic pacers, it is important to ascertain that the stimulus is having the desired effect. Pulse generation which causes contraction of the selected chamber is termed "capture", and the method of determining that the pacer stimuli are achieving capture is called "capture verification". Capture verification techniques are based on detecting the potential evoked when the heart is captured. If there is no capture, there is no evoked potential, and the amplitude and/or duration of the stimulating pulse must then be adjusted to assure consistent capture. It follows that each time the heart is paced, the cardiac electrical activity may be monitored to detect the presence of the evoked potential and thereby verify capture.

In practice, however, capture verification is fraught with problems, one of the more significant being of a signal-to-noise nature in which the signal sought to be detected is masked by after-potentials attributable to electrode polarization. After the stimulating pulse is delivered, the electrode must "settle down" to allow detection of the evoked potential indicative of capture. This requires a suitable period of delay, which itself precludes the desired detection. Accordingly, some capture verification techniques seek to filter the signal from the masking after-potential, necessitating additional circuitry and space.

Accordingly, the invention provides a cardiac pacing lead assembly, comprising an electrode having an exposed surface layer of a metal oxide situated to be in electrically coupled relationship with excitable cardiac tissue of the heart when said lead assembly is implanted in a patient, for stimulating the cardiac tissue when said electrode is electrically energized and for sensing electrical activity of the heart in response to the stimulation when said electrode is de-energized, and an electrical conductor electrically connected at one end to said electrode for delivering electrical energization to the electrode and for conducting signals representing said electrical activity from the electrode, and having a terminal at its other end for connection to electrical generating and detecting circuitry of a cardiac pacemaker, characterized in that said metal oxide is iridium oxide.

The invention also provides a cardiac pacemaker for stimulating and sensing electrical activity of a human heart, comprising a pulse generator, a pacing lead assembly comprising an electrode having a surface layer of a metal oxide situated to electrically interact with excitable cardiac tissue of the heart when said lead assembly is implanted in a human patient and a stimulating pulse is applied thereto, and a conductor having a distal end electrically connected to said electrode and a proximal end electrically connected to said pulse generator for applying stimulating pulses from said pulse generator to said electrode, characterized in that said metal oxide is iridium oxide.

According to the present invention, an iridium oxide layer is formed on the surface of the stimulating electrode to provide a considerable reduction in the stimulation threshold as compared to electrodes and electrode materials and compositions previously employed in the cardiac pacing field. It appears that the lower threshold achieved with the iridium oxide coating may also result in a significant reduction of injury to the myocardial tissue at the stimulation site. Moreover, iridium oxide appears to possess greater physical integrity and superior charge transfer capability per unit area than materials heretofore commonly employed for pacing electrodes, including specialized coatings such as platinum black.

Previously, it had been found and reported that iridium oxide films exhibit electrochromic behavior, which led to the use of iridium oxide electrodes in electrochromic displays (e.g., see Dautremont-Smith et al., "Electrochromic Cells with Iridium Oxide Display Electrodes", Solid State Ionics 2 (1981) pp. 13-18). Such iridium oxide films have been produced by cyclic anodic growth on an iridium substrate, or by complete anodization of thin iridium films, referred to by the acronym AIROF (for anodic iridium oxide film). More recently, iridium oxide films have been produced by direct deposition on selected substrates through reactive sputtering from an iridium target, referred to as SIROF (for sputtered iridium oxide film).

Iridium oxide electrodes have heretofore been used in certain medical applications, such as for measuring tissue impedances (e.g., see Gielen et al., "Comparison of electrode impedances of Pt, PtIr (10% Ir) and Ir-AIROF electrodes used in electrophysiological experiments", Medical and Biological Engineering & Computing, January 1982, pp. 77-83): for measuring acidity in the upper gastro-intestinal tract (e.g., see Papeschi et al., "The iridium/iridium oxide electrode for in vivo measurement of oesophagael and gastric pH", Journal of Medical Engineering and Technology, Vol.8, No. 5, Sep.-Oct. 1984, pp. 221-223); and for measuring acidity changes in the blood (e.g., see Papeschi et al., "An iridium/iridium oxide electrode for in vivo monitoring of blood pH changes". Journal of Medical engineering and Technology, Vol. 5, No. 2, Mar. 1981. pp. 86-88, and Cammilli et al., "Preliminary Experience with the pH-triggered Pacemaker", PACE. Vol. 1, Oct.-Dec. 1978, pp. 448-457). In the Cammilli et al. publication, the authors reported on the use of an iridium oxide electrode for continuous in vivo detection of variations of mixed venous blood pH. According to the article, a rapid decrease of blood pH was utilized as a measure of variation of the patient's metabolic rate and employed to produce an appropriate variation in the stimulation rate for physiological pacing.

Such reports neither teach nor suggest using an iridium oxide electrode for stimulating or sensing electrical activity of the heart. Indeed, in the medical applications of iridium oxide electrodes previously reported, any stray electrical signals would have been deemed as interfering with and undesirable to the purpose for which the electrodes were being used.

Although iridium oxide electrodes have been used more recently in electrophysiological experiments, such as for neuroelectrical experimentation with brain activity in small animals, the proposal for such use was attributable to an absolute requirement for extremely fine electrode wires, with active surface areas on the order of 20 square microns. It had been found that even platinum electrodes of such tiny size disintegrated on the passage therethrough of relatively low levels of current. It was found that an iridium oxide coating was capable of withstanding the necessary current without significant deterioration. In contrast to the relatively tiny surface areas of concern in these physiological experiments, electrodes for the stimulation of excitable heart tissue in artificial cardiac pacing, or for the detection of cardiac electrical activity, require considerably greater surface areas.

The present invention recognizes the extraordinary capability of iridium oxide to perform as a charge flow transducer between media exhibiting different charge flow mechanisms, and, despite its relatively inferior characteristics as an electrical conductor compared to conventional pacing electrode materials, that certain properties of iridium oxide make it particularly effective for pacing electrodes, both for stimulating and sensing electrical activity of the heart. This appears to arise, in part, from the two basic mechanisms for current flow across a pacing electrode. One is the purely capacitive mechanism by which electron flow away from the cathode causes electrical charges in the solution at the electrode-electrolyte interface to orient themselves such that a displacement current occurs through the electrolyte, i.e., because the electrolyte is an ionic medium, the slight displacement of the ions in reorientation creates a charge flow. When the electrical potential across the electrode-electrolyte interface is sufficiently large, chemical reactions begin to occur and current flows. At that point, the mechanism is no longer capacitive. With conventional electrode materials, the chemical reactions are substantially irreversible.

Iridium oxide demonstrates a capacity to readily accept electrons out of an electrolytic solution, and thus can operate as a highly efficient transducer between an electron flow conductor -- such as a metal electrode -- and an ionic flow conductor -- such as the saline fluid of the body.

Iridium oxide may be deposited as a relatively thick porous layer on a metal substrate for use as a pacing electrode. The porous structure accommodates water from the body saline. In a typical reaction involving a conventional electrode, a negative potential on the electrode repels electrons, and hydrogen is released from the water in the process. In contrast, with an iridium oxide layer relatively tiny potential differences across the electrode-electrolyte interface are effective to produce the reactions and consequent current flow, while the pores trap the reaction products that would otherwise diffuse away and might injure tissue in the vicinity of the stimulation site. More importantly, with the iridium oxide electrode the reactions are reversible upon reversal of the voltage.

A capacitive effect occurs with an iridium oxide coated electrode, but to a considerably lesser extent than that occurring, for example, with a platinum electrode. Rather, the interface across the iridium oxide surface appears to be primarily resistive. Accordingly, an iridium oxide coated pacing electrode exhibits lower polarization than is observed with conventional pacing electrodes; which is to say that the voltage buildup at the interface is smaller for a given charge flow through the iridium oxide electrode. Hence, more energy is available for tissue stimulation. Furthermore, the low polarization and resultant relatively small voltage buildup at the interface allows detection of cardiac electrical activity relatively quickly after stimulation, in use of the iridium oxide coated electrode for stimulation and sensing, and allows reliable capture verification without the need for special filters or other apparatus beyond a simple detection circuit.

The reasons for the highly efficient behavior of iridium oxide as a charge flow transducer between media exhibiting different charge flow mechanisms are not fully understood. In further part, it appears to be attributable to the numerous oxidation states within a film of the material. These oxidation states seem to be relatively stable, with low activation energies, and, therefore, the layer tends to perform more as a resistor than a capacitor. The result is that current flow is facilitated, but without the buildup of residual voltages. Again, the virtual absence of residual voltages serves to eliminate the masking problem and allow reliable capture verification.

In a preferred embodiment of the invention, the iridium oxide layer is confined to the surface of a tip electrode adapted to be positioned in electrically stimulating relationship with the excitable myocardial tissue at a preselected stimulation site in a desired chamber on the right side of the heart. The underlying electrode may be composed of any conventional material for pacing electrode applications, such as titanium, and is preferably but not necessarily a porous structure. The electrode is electrically connected to the conductive coil within the lead. The anode may also be coated with iridium oxide, and, for bipolar stimulation, may be a ring electrode electrically insulated from the cathode and electrically connected to a second conductive coil within the lead, or for unipolar stimulation, may simply be an iridium oxide coated button or foil conductively affixed to the pulse generator case.

In alternative embodiments of the invention, recesses are provided in the surface of the underlying electrode substrate, in which the iridium oxide coating is confined. The depth of the recesses and the thickness of the iridium oxide layer therein may be controlled such that the exposed surface of the IrO layer is recessed from the outermost surface of the electrode tip. The current density is greater along the iridium oxide regions, and, since they are slightly removed from direct contact with excitable heart tissue, the tissue is less likely to suffer from abrasion or localized pH changes at the electrode-electrolyte interface. In these alternative embodiments, the tip electrode may be substantially spherical in shape, and the recesses may be dimples or circumferential grooves in the surface of the sphere. The anodic electrode for bipolar stimulation may have a rippled surface with an iridium oxide coating in the trough of the ripples.

Accordingly, primary objects of the present invention include the provision of (1) an improved pacing electrode with a metal oxide surface for stimulating or sensing electrical activity of the heart; (2) a low threshold, cardiac tissue stimulating electrode with a tip portion having a metal oxide coating thereon to achieve low polarization, with maximum reversibility of surface reactions, and less injury to tissue in the vicinity of the stimulation site; (3) an improved pacing electrode having a surface layer that performs highly efficient transduction between the electron flow in the underlying metallic substrate and the ionic current flow in the electrolyte medium of the body; and (4) a pacemaker electrode for both stimulating and sensing electrical activity of the heart, which provides low polarization and little voltage buildup to preclude masking the evoked potential on stimulation and, thereby, allows relatively rapid sensing of that potential to reliably verify capture.

Prior art electrodes for use in medical stimulation are known from DE-A-2056493 and EP-A-0126981. DE-A-2056493 discloses a cardiac pacing lead assembly as described in the preamble of claim 1. EP-A-0126981 discloses a low-polarisation low-threshold electrode for use in medical stimulation leads. It provides one solution to object (4) above.

The above and still further objections, features, aspects and advantages of the present invention will become apparent to those of ordinary skill in the field to which the invention pertains from a consideration of the following detailed description of certain preferred embodiments, taken in conjunction with the accompanying drawings, in which:
FIG. 1 is a simplified cross-sectional view of a pacing electrode assembly according to the invention, taken along the axis of the configuration, which is circular in transverse cross-section;
FIG. 2 is a simplified representation of an alternative embodiment of a pacing electrode assembly as part of a lead assembly arranged for unipolar stimulation, in a cardiac pacemaker implanted in the body:
FIGS. 3, 4, 5, and 6 are perspective views of alternative embodiments of a stimulating tip electrode;
FIG. 7 is a perspective view of an alternative embodiment of an anodic ring electrode; and
FIGS. 8 and 9 are electrograms taken from test dogs, respectively using conventional electrodes and iridium oxide coated electrodes for stimulation and sensing, in which the top portion of each FIG. represents a surface electrogram and the bottom portion an electrogram taken between the indifferent electrode and the tip electrode of an implanted lead assembly.

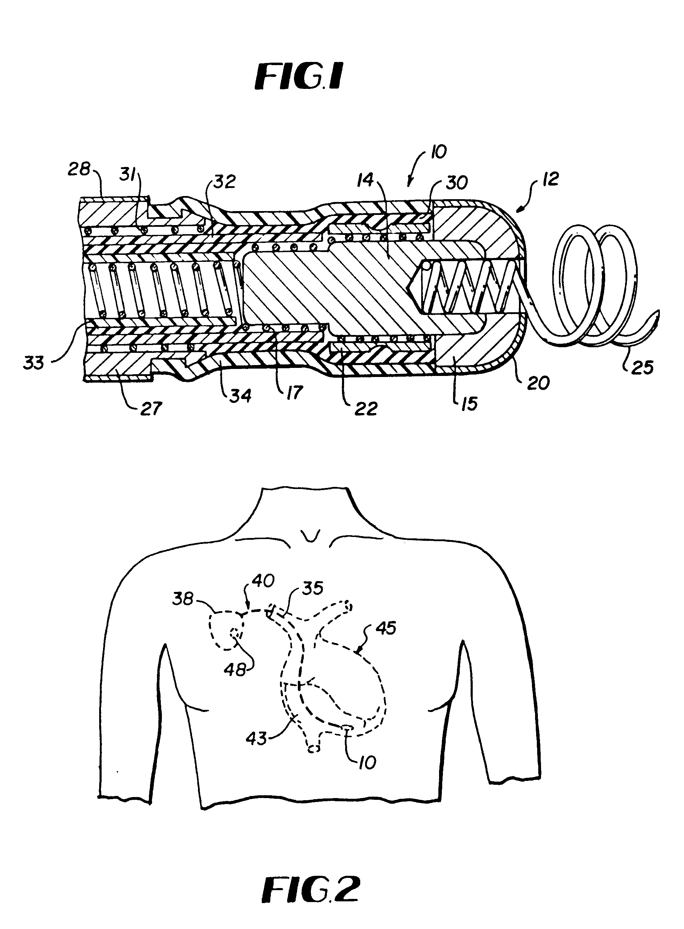

Referring now to FIG. 1, electrode assembly 10 is part of and located at the distal end of a pacing lead assembly (to be described more fully in connection with FIG. 2). The proximal end of the lead assembly is conventionally arranged for connection to the pulse generator of an implantable cardiac pacemaker. The electrode assembly shown in FIG. 1 is a simplified depiction since there is no need to illustrate those details of electrode structure which are well known.

Assembly 10 is configured for endocardial positioning, in which tip electrode (cathode) 12 is adapted to be placed in electrically stimulating relationship with excitable cardiac tissue within a selected chamber of the heart. Substrate 15 of tip 12, and integral stem 14, are composed of any conventional electrode materials, such as platinum, platinum-iridium alloy, iridium, tantalum, or titanium, by way of example; and preferably, titanium. A coil 17 of electrically conductive wire within the lead assembly is maintained in solid electrical contact with tip 12 by means of a metal sleeve 22 crimping the coil against the stem. A corkscrew 25 may be affixed to the electrode assembly in a conventional manner to provide active fixation of the stimulating electrode to the myocardium after the electrode has been positioned properly in the selected chamber.

The surface of the cathodic tip electrode 12 is coated with a film or layer 20 of iridium oxide, which may be an AIROF, SIROF, TIROF (thermal iridium oxide film), or formed in any other suitable manner. The specific process by which the electrode substrate is coated constitutes no part of the present invention. The iridium oxide layer may have a thickness of approximately 200 nanometers, although any layer thickness exceeding about 100 nanometers appears to be satisfactory to obtain the desirable results, with air exposed surface area of approximately 8.5 square millimeters. Preferably, the substrate 15 of tip 12 has a porous surface structure, such that the iridium oxide coating assumes the lacework contour of the surface and promotes ingrowth of cardiac tissue to reduce abrasion of the adjacent tissue.

Stem 14 and substrate 15 may be formed integrally or separately (in the latter case, the two are then pressed together and bonded) by conventional powder metallurgy process, in which powdered titanium is packed into a mold, compressed, and thereafter sintered at a temperature and for a time sufficient to cause partial melting into a relatively porous electrically conductive structure.

An exemplary preferred process for forming a TIROF film on porous titanium tip electrode substrates is as follows. The electrode tips are etched in hot 10% oxalic acid, 100°C for 30 minutes; thereafter rinsed in distilled water and placed in an iridium solution with only the tip portions to be coated contacted by the solution. The Ir solution is prepared by dissolving 0.4 gram IrCl₃-3H₂O in 10 ml 20% HCl, heating the solution to evaporate the HCl down to one-quarter volume and restoring the original volume with absolute isopropanol, the resulting solution to be used within 7 to 14 days. Following a 16 hour soak in this solution, the electrodes are dried at room temperature for one hour, and then annealed at 320°C for another hour. The steps of soaking, drying and annealing are repeated, and the electrodes are then annealed again at 320°C for a period of from 3 to 6 hours. The foregoing and other processes described herein for forming the iridium oxide layer on electrode substrates does not constitute a part of the present invention.

In an exemplary SIROF process, the electrode substrate may be reactively coated with iridium oxide in a conventional diode RF sputtering system. The substrate is initially positioned and maintained in good thermal contact with the water cooled platform of the sputtering system. Any portion of the surface which is not to be coated is suitably masked. Pre-sputtering is performed with an iridium target in pure oxygen at an ambient pressure of about 20 µm (microns) for approximately 20 minutes to one-half hour. The pressure is then reduced to the range from about 2 to 4 µm (microns), and sputtering is performed with a target power density of about 0.6 to 0.8 watt per square centimeter. The process is continued until an iridium oxide layer of the desired thickness is deposited, about three hours.

For bipolar stimulation, the electrode assembly includes an anodic electrode 27, preferably of titanium, configured as a ring electrode insulatively spaced behind tip 12 by a sufficient distance to avoid the shunting of current between the edges of the two electrodes. The anode also may be coated with a layer 28 of iridium oxide at its exposed surface, in the same manner as cathodic electrode tip 12. A second coil 31 of conductive wire is maintained in electrical connection with the interior of anode 27 by confining the coil, for example, between the anode and a metal ring (not shown) at the far end of the anode. Coil 31 is part of the lead assembly, and is arranged via a connector (not shown) at the proximal end for coupling the anode to a point of reference potential at the pulse generator. An electrically insulating mass 30 of silicone rubber may be used to encapsulate the internal elements of the electrode assembly, including polyurethane sleeves 32 and 33, and an outer polyurethane sleeve 34 covers the assembly from cathode tip 12 to anode 27 leaving the IrO surfaces of those two electrodes exposed.

Referring now to FIG. 2, a pacing lead assembly 35 includes electrode assembly 10 at its distal end and is connected at its proximal end to appropriate points of electrical potential of the conventional circuitry, including the pulse generator, housed within a metallic case 38. The combination of the circuitry in case 38 and the pacing lead assembly 35 constitutes cardiac pacemaker 40. As shown in FIG. 2, the pacing lead assembly 35 is inserted transvenously until the iridium oxide coated cathodic tip is properly positioned in contact with or adjacent to excitable tissue within the selected chamber; in this example, the right ventricle 43 of the patient's heart 45. Case 38 houses a pulse generator, a detection circuit, the batteries, and other conventional electronic circuitry, and includes an electrical connector mating with the connector at the proximal end of the pacing lead assembly. In practice, the case is implanted in a surgical incision which forms a subcutaneous pouch in the patient's chest, after connection to the lead assembly.

The pacing lead assembly 35 shown in FIG. 2 may he arranged for unipolar stimulation, with the case 38 or a limited region 48 thereof comprising an iridium oxide-coated foil being used as the anode. Of course, in that situation the anodic ring and associated coil of the electrode assembly shown in FIG. 1 would not be present. Region 48 may include a substrate of iridium foil which has been anodized to form an AIROF film thereon, and the uncoated side of the foil then conductively bonded to titanium case 38. Alternatively, region 48 may comprise a titanium button on which an iridium oxide layer, preferably having a thickness exceeding 100 nanometers, is formed as described earlier herein.

In an exemplary preparation of an AIROF electrode, the iridium foil is cleaned and polished ultrasonically in an ethanol bath, followed by etching of the foil surface by immersion in mild saline solution and applying a sinusoidal voltage of 15 volts at 10 Hz between the foil and a reference electrode (such as platinum) for a period of about 60 seconds. An iridium oxide layer is then grown on the etched iridium foil by cyclic anodization of the foil at room temperature in an electrolyte consisting of a mild solution of sulfuric acid. The anodization is carried out by application of a triangular voltage between the foil and the electrolyte, of between +0.25V and -1.25V as measured with a calomel electrode, for a period of time sufficient to produce an iridium oxide layer of the desired thickness. One side of the foil is then cleaned and bonded to case 38 to provide a strong electrical connection therebetween.

In operation of the pacemaker of FIG. 2, stimulating pulses delivered by the pulse generator to the cathodic electrode cause an electric field to be impressed on the myocardial tissue at the cathode site. If the field strength and current density of the electric field is sufficient to reach or exceed the stimulation threshold, capture is achieved. The efficient translation of the iridium oxide layer on the cathode tip results in considerably lower stimulation thresholds and electrode polarization than may be achieved with pacing electrodes composed of materials heretofore utilized for such applications. Acute stimulation thresholds as low as approximately 0.2 volt have been observed in pacing experiments on test dogs using lead assemblies with iridium oxide coated cathodes.

A stimulating pulse is delivered by the pulse generator to the heart through the circuit which includes the lead, the cathodic electrode, the anodic electrode, the body tissue and fluid. The events leading up to the pacing depend upon the particular type of pacemakers but in general the stimulating pulse is of relatively short duration, e.g., 0.5 ms, lasting for the period of closure of a switch (typically, an NMOS FET) to discharge the main capacitor through a smaller coupling capacitor. The latter is charged in the process, and it is customary to actively discharge the coupling capacitor when the aforementioned switch is opened, by closing another switch (typically, a PMOS FET) to provide a reverse current path for an interval of about 10 ms. The sense amplifier is unhooked during stimulation and throughout the active discharge interval, but thereafter receives signals representing electrical activity sensed by the tip electrode (cathode). With conventional pacing electrodes, electrode polarization may result in a lingering after-potential following delivery of each pacing pulse. The after-potential may continue for hundreds of milliseconds, and, if it extends beyond the refractory period, may easily result in false detection as a cardiac event. In contrast, the low polarization iridium oxide coated pacing electrodes of the present invention virtually eliminate after-potentials, and thereby allow sensing of evoked potentials and other valid cardiac events within a relatively short time after stimulation, e.g., approximately 25 ms and consistently within the first 100 milliseconds.

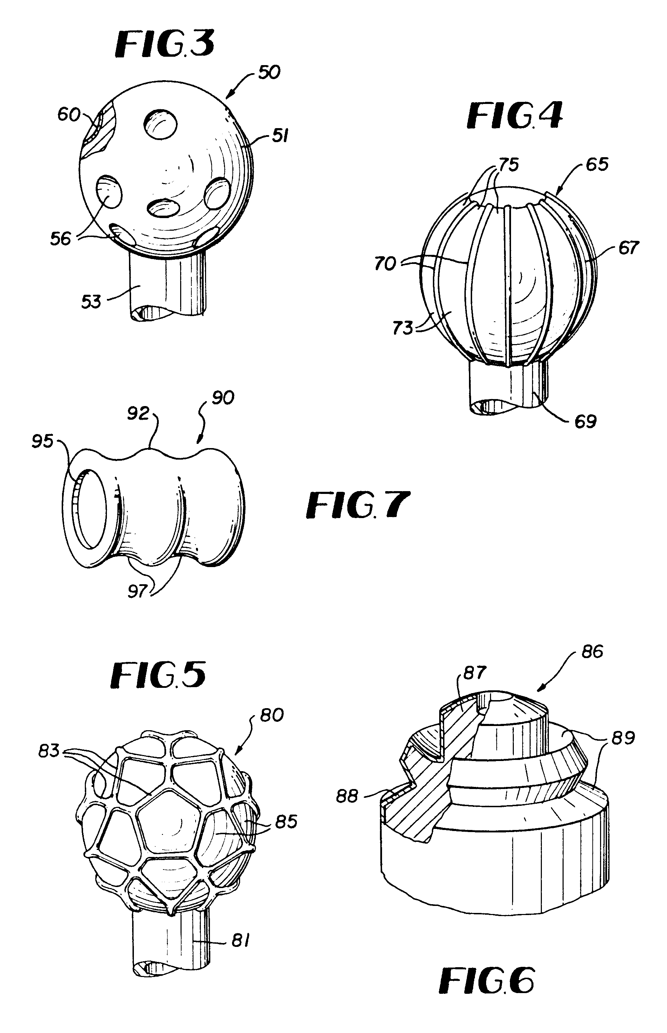

FIGS. 3, 4, 5 and 6 show various alternative embodiments of stimulating cathodic electrodes in which the iridium oxide layer may be confined to recesses in the electrode surface. Referring to FIG. 3, stimulating cathode 50 comprises a spherical tip 51 a tubular shank 53, each of which is composed of titanium. Tip 51 has a plurality of recesses in a regular pattern of dimples 56 on its outer surface, and the iridium oxide layer 60 may be confined to the dimples. In the embodiment of FIG. 4, electrode 65 includes spherical tip 67 and cylindrical shank 69, the tip having a plurality of spaced circumferential ribs 70 lying at its surface and defining a plurality of recessed regions 73 covered with iridium oxide film 75.

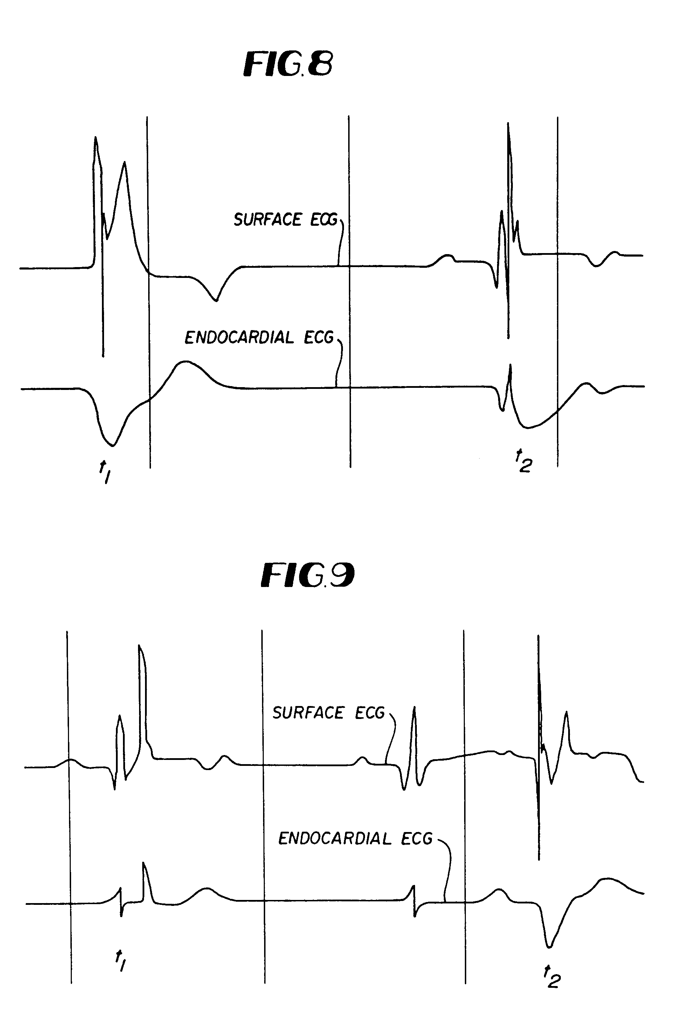

In the embodiment of FIG. 5, the spherical tip 80 is conductively connected to stem 81 and has a regular pattern of polygonal ribs 83 enclosing recesses coated with an iridium oxide layer 85. Referring now to FIG. 6, another embodiment of a stimulating cathodic electrode comprises a tip portion 86 having a substrate 87 and an iridium oxide layer 88 overlying the substrate. In this embodiment, the electrode substrate is of generally hemispherical shape with grooves 89 cut in spaced concentric rings each sharing the longitudinal axis of the tip portion.

In each of the embodiments of FIGS. 3-6, the electrode substrate is preferably titanium, and an iridium oxide layer may be formed as a TIROF by the exemplary process described earlier herein. The IrO layer may be confined to the recesses in each case by polishing the raised portion of the surface after forming the layer. If the coated surface is porous, the iridium oxide layer will conform generally to its contour and thereby maintain the desired porosity.

Referring now to FIG. 7, another embodiment of an anodic electrode for bipolar stimulation comprises ring anode 90 having a tubular configuration with rippled surface 92 and an iridium oxide layer 95 overlying the troughs of the ripples. The thickness of layer 95 is preferably about 200 nanometers.

Referring now to FIGS. 8 and 9, each of these FIGS. shows an upper trace of a surface ECG, and a lower trace of an endocardial ECG taken across the the indifferent electrode and the stimulating cathodic electrode. The cathode was used for stimulation, and for sensing after the application of stimuli and at all other times. The traces in FIG. 8 were obtained from a test dog in which a lead assembly with a conventional platinum-iridium stimulating electrode was implanted. It will be observed that in the lower trace the two waveforms are confusingly similar, and indeed, appear to indicate capture at both times t₁ and t₂. However, the surface electrogram of the upper trace clearly indicates a pace with captured QRS at time t₁, and a P-wave and QRS complex with a pacing pulse at time t₂ but at that point the tissue is depolarized so there is no capture. In the latter instance it was the after-potential on the electrode that was detected. Although the traces of FIG. 8 visually allow the trained observer to distinguish between capture and non-capture, the distinction may not be readily detected by conventional electronic detection circuitry, particularly where, as here, the sensed waveforms are confusingly similar. For example, the waveform at time t₂ in the lower trace of FIG. 8 would be detected as capture by a typical level detector.

The traces in FIG. 9 were obtained from experiments on a test dog in which the implanted lead assembly was provided with an iridium oxide coated stimulating electrode. It will be observed here that the lower trace indicates non-capture at time t₁ and capture at time t₂, and that the two waveforms are clearly distinguishable by detection circuitry as well. The first sensed event is virtually a straight line which will produce no reading, whereas the second sensed event will be detected as capture.

## Claims

1. A cardiac pacing lead assembly (35), comprising an electrode (12) having an exposed surface layer (20) of a metal oxide situated to be in electrically coupled relationship with excitable cardiac tissue of the heart (45) when said lead assembly is implanted in a patient, for stimulating the cardiac tissue when said electrode is electrically energized and for sensing electrical activity of the heart in response to the stimulation when said electrode is de-energized, and an electrical conductor (17) electrically connected at one end to said electrode for delivering electrical energization to the electrode and for conducting signals representing said electrical activity from the electrode, and having a terminal at its other end for connection to electrical generating and detecting circuitry of a cardiac pacemaker (40), characterized in that said metal oxide is iridium oxide.

2. The pacing lead assembly (35) of Claim 1, in which the iridium oxide surface layer (20) has a thickness and an exposed surface area of sufficiently low polarization to resist buildup of residual voltage and thereby to enable the electrode (12) to sense electrical activity of the heart (45) within less than about 100 milliseconds after being de-energized, to verify capture from the evoked potential.

3. The pacing lead assembly (35) of any of the preceding claims, in which the surface of the electrode (12) is porous and said iridium oxide surface layer (20) follows the interstices of the porous surface.

4. The pacing lead assembly (35) of any of the preceding claims, in which said iridium oxide surface layer (20) has a thickness exceeding 100 nanometers.

5. The pacing lead assembly (35) of any of the preceding claims, further including a second electrode (27) having an iridium oxide layer (28) on its surface, the second electrode being insulatively spaced-apart from and in fixed relationship with the first-mentioned electrode (12) for bipolar stimulation of the patient's heart (45), and a second electrical conductor (31) electrically connected at one end to said second electrode and having a terminal at its other end for connection to a point of reference potential of the circuitry.

6. A cardiac pacemaker (40) for stimulating and sensing electrical activity of a human heart (45), comprising a pulse generator, a pacing lead assembly (35) comprising an electrode (12) having a surface layer (20) of a metal oxide situated to electrically interact with excitable cardiac tissue of the heart when said lead assembly is implanted in a human patient and a stimulating pulse is applied thereto, and a conductor (17) having a distal end electrically connected to said electrode and a proximal end electrically connected to said pulse generator for applying stimulating pulses from said pulse generator to said electrode, characterized in that said metal oxide is iridium oxide.

7. The cardiac pacemaker (40) of claim 6, further including a metal case (38) housing the pulse generator, and an indifferent electrode (48) affixed to said case and having a surface layer of iridium oxide thereon, for use in unipolar stimulation.

8. The cardiac pacemaker (40) of claim 6, further including a detection circuit, and wherein the iridium oxide surface layer (20) of the electrode (12) provides rapid recovery from after-potentials at said layer upon cessation of the stimulating pulse to enable sensing of the potential evoked and verification of capture of the heart by said stimulating pulse, and said conductor (17) further having its proximal end electrically connected to said detection circuit for applying evoked potentials sensed by said electrode (12) thereto.

9. The cardiac pacemaker (40) of claim 8, wherein said rapid recovery takes place less than 100 milliseconds after cessation of the stimulating pulse.

## Patentansprüche

1. Herzstimulierungs-Leitungsanordnung (35), mit einer Elektrode (12) mit einer exponierten Oberflächenschicht (20) aus einem Metalloxid, die derart angeordnet ist, daß sie in elektrisch gekoppelter Beziehung mit erregbarem Herzgewebe des Herzens (45) ist, wenn die Leitungsanordnung in einem Patienten implantiert ist, zum Stimulieren des Herzgewebes, wenn die Elektrode elektrisch erregt wird und zum Abfühlen elektrischer Aktivität des Herzens als Reaktion auf die Stimulation, wenn die Elektrode aberregt wird, sowie mit einem elektrischen Leiter (17), der an einem Ende zur Abgabe elektrischer Energie an die Elektrode und zum Leiten von die elektrische Aktivität darstellenden Signalen von der Elektrode elektrisch mit der Elektrode verbunden ist und einen Anschluß an seinem anderen Ende zur Verbindung mit einer elektrischen Generator- und Detektorschaltung eines Herzschrittmachers (40) aufweist, dadurch gekennzeichnet, daß das Metalloxid Iridiumoxid ist.

2. Stimulations-Leitungsanordnung (35) nach Anspruch 1, bei der die Iridiumoxid-Oberflächenschicht (20) eine Dicke und eine exponierte Oberfläche von ausreichend niedriger Polarisation aufweist, um dem Aufbau von Restspannung zu widerstehen und es dadurch der Elektrode (12) zu ermöglichen, elektrische Aktivität des Herzens (45) innerhalb weniger als etwa 100 Millisekunden nach ihrer Aberregung abzufühlen, zur Verifizierung des Einfangens aus dem hervorgerufenen Potential.

3. Stimulations-Leiteranordnung (35) nach einem der vorhergehenden Ansprüche, bei der die Oberfläche der Elektrode (12) porös ist und die Iridiumoxid-Oberflächenschicht (20) den Zwischenräumen der porösen Oberfläche folgt.

4. Stimulations-Leiteranordnung (35) nach einem der vorhergehenden Ansprüche, bei der die Iridiumoxid-Oberflächenschicht (20) eine Dicke von mehr als 100 Nanometern aufweist.

5. Stimulations-Leiteranordnung (35) nach einem der vorhergehenden Ansprüche, weiterhin enthaltend eine zweite Elektrode (27) mit einer Iridiumoxid-Schicht (28) auf ihrer Oberfläche, wobei die zweite Elektrode einen isolierenden Abstand von der ersterwähnten Elektrode (12) aufweist und in fester Relation zu dieser für eine bipolare Stimulation des Herzens (45) des Patienten steht, sowie einen zweiten elektrischen Leiter (31), der an einem Ende elektrisch mit der zweiten Elektrode verbunden ist und einen Anschluß an seinem anderen Ende zur Verbindung mit einem Punkt mit einem Bezugspotential der Schaltung aufweist.

6. Herzschrittmacher (40) zum Stimulieren und Abfühlen der elektrischen Aktivität eines menschlichen Herzens (45), enthaltend einen Impulsgenerator, eine Stimulations-Leiteranordnung (35) mit einer Elektrode (12) mit einer Oberflächenschicht (20) aus einem Metalloxid, die derart angeordnet ist, daß sie elektrisch mit erregbarem Herzgewebe des Herzens zusammenwirkt, wenn die Leiteranordnung in einem menschlichen Patienten implantiert ist und ein Stimulationsimpuls an sie angelegt wird, sowie einen Leiter (17) mit einem elektrisch mit der Elektrode verbundenen distalen Ende und einem elektrisch mit dem Impulsgenerator verbunden proximalen Ende zum Anlegen von Stimulationsimpulsen aus dem Impulsgenerator an die Elektrode, dadurch gekennzeichnet, daß das Metalloxid Iridiumoxid ist.

7. Herzschrittmacher (40) nach Anspruch (6), weiterhin enthaltend ein den Impulsgenerator beherbergendes Metallgehäuse (38) und eine an dem Gehäuse angebrachte indifferente Elektrode (48) mit einer Oberflächenschicht von Iridiumoxid, zur Verwendung bei der unipolaren Stimulation.

8. Herzschrittmacher (40) nach Anspruch 6, weiterhin enthaltend eine Detektorschaltung und wobei die Iridiumoxid-Oberflächenschicht (20) der Elektrode (12) für eine schnelle Erholung von Nachpotentialen bei dieser Schicht nach Aufhören den Stimulationsimpulses sorgt, um das Abfühlen des hervorgerufenen Potentials und die Verifikation des Einfangens des Herzens durch den Stimulationsimpuls zu ermöglichen, wobei weiterhin das proximale Ende des Leiters (17) elektrisch mit der Detektorschaltung zum Anlegen von hervorgerufenen von der Elektrode (12) abgefühlten Potentialen an diese verbunden ist.

9. Herzschrittmacher (40) nach Anspruch 8, bei dem die schnelle Erholung weniger als 100 Millisekunden nach dem Aufhören des Stimulationsimpulses stattfindet.

## Revendications

1. Un ensemble connecteur pour stimulation cardiaque (35), comprenant une électrode (12) comportant une couche superficielle exposée (20) formée d'un oxyde métallique et située de façon à être en position de couplage électrique avec un tissu cardiaque excitable du coeur (45) quand ledit ensemble connecteur est implanté dans un patient, afin de stimuler le tissu cardiaque quand ladite électrode est excitée électriquement et afin de détecter l'activité électrique du coeur en réponse à la stimulation lorsque l'électrode est désexcitée, ainsi qu'un conducteur électrique (17) relié électriquement par une extrémité à ladite électrode, afin de produire une excitation électrique de cette dernière et de transmettre des signaux représentant ladite activité électrique à partir de l'électrode, et comportant à son autre extrémité une borne pour établir une connexion avec un circuit de génération et détection de courant électrique d'un stimulateur cardiaque (40), caractérisé en ce que ledit oxyde métallique est de l'oxyde d'iridium.

2. L'ensemble connecteur de stimulation (35) selon la revendication 1, dans lequel la couche superficielle d'oxyde d'iridium (20) a une épaisseur et une aire de surface exposée de polarisation suffisamment faible pour s'opposer à l'accumulation d'une tension résiduelle et pour permettre ainsi à l'électrode (12) de détecter l'activité électrique du coeur (45) en moins d'environ 100 millisecondes après avoir été désexcitée, afin de vérifier une "contraction cardiaque" à partir du potentiel engendré.

3. L'ensemble connecteur de stimulation (35) selon l'une quelconque des revendications précédentes, dans lequel la surface de l'électrode (12) est poreuse et ladite couche superficielle (20) d'oxyde d'iridium suit les interstices de la surface poreuse.

4. L'ensemble connecteur de stimulation (35) selon l'une quelconque des revendications précédentes, dans lequel ladite couche superficielle (20) d'oxyde d'iridium a une épaisseur supérieure à 100 nanomètres.

5. L'ensemble connecteur de stimulation (35) selon l'une quelconque des revendications précédentes, comprenant en outre une seconde électrode (27) comportant une couche d'oxyde d'iridium (28) sur sa surface, la seconde électrode étant espacée de façon isolante de l'électrode (12) mentionnée en premier, et étant située dans une relation fixée par rapport à celle-ci, pour une stimulation cardiaque du coeur (45) du patient, ainsi qu'un second conducteur électrique (31) relié électriquement par une extrémité à ladite seconde électrode et pourvu à son autre extrémité d'une borne pour établir une connexion avec un point de potentiel de référence du circuit.

6. Un stimulateur cardiaque (40) pour une stimulation et une détection d'activité électrique d'un coeur humain (45), comprenant un générateur d'impulsions, un ensemble conrecteur de stimulation (35) comprenant une électrode (12) comportant une couche superficielle (20) formée d'un oxyde métallique et située de façon à produire une interaction électrique avec un tissu cardiaque excitable du coeur quand ledit ensemble connecteur est implanté dans un patient humain et quand une impulsion de stimulation lui est appliquée, et un conducteur (17), comportant une extrémité distale reliée électriquement à ladite électrode et une extrémité proximale reliée électriquement audit générateur d'impulsions pour appliquer des impulsions de stimulation provenant dudit générateur à ladite électrode, caractérisé en ce que ledit oxyde métallique est de l'oxyde d'iridium.

7. Le stimulateur cardiaque (40) selon la revendication 6, comprenant en outre un boîtier métallique (38) contenant le générateur d'impulsions et une électrode indifférente (48) fixée sur ledit boîtier et portant une couche superficielle d'oxyde d'iridium, pour utilisation en stimulation unipolaire.

8. Le stimulateur cardiaque (40) selon la revendication 6, comprenant en outre un circuit de détection, et dans lequel la couche superficielle (20) d'oxyde d'iridium de l'électrode (12) produit une récupération rapide à partir de potentiels rémanents dans ladite couche lors d'un arrêt de l'impulsion de stimulation afin de permettre une détection du potentiel engendré et une vérification de la contraction du coeur par ladite impulsion de stimulation, et ledit conducteur (17) ayant en outre son extrémité proximale qui est reliée audit courant de détection pour lui appliquer les potentiels engendrés qui ont été détectés par ladite électrode (12).

9. Le stimulateur cardiaque (40) selon la revendication 8, dans lequel ladite récupération rapide se produit en moins de 110 millisecondes après cessation de l'impulsion de stimulation.
